(19) Europäisches Patentamt
European Patent Office
Office européen des brevets

(11) Veröffentlichungsnummer : **0 006 613 B2**

# (12) NEUE EUROPÄISCHE PATENTSCHRIFT

(45) Veröffentlichungstag der neuen Patentschrift : **26.08.92 Patentblatt 92/35**

(51) Int. Cl.$^5$ : **C07C 233/05**

(21) Anmeldenummer : **79102131.4**

(22) Anmeldetag : **27.06.79**

## (54) Verfahren zur Herstellung von N,N'-Diacetylethylendiamin.

(30) Priorität : **30.06.78 DE 2828765**

(43) Veröffentlichungstag der Anmeldung : **09.01.80 Patentblatt 80/01**

(45) Bekanntmachung des Hinweises auf die Patenterteilung : **16.09.81 Patentblatt 81/37**

(45) Bekanntmachung des Hinweises auf die Entscheidung über den Einspruch : **26.08.92 Patentblatt 92/35**

(84) Benannte Vertragsstaaten : **BE DE FR GB IT NL**

(56) Entgegenhaltungen :
**DE-B- 2 118 282**
**DE-B- 2 133 458**
**SU-A- 180 605**

(73) Patentinhaber : **HOECHST AKTIENGESELLSCHAFT**
**Postfach 80 03 20**
**W-6230 Frankfurt am Main 80 (DE)**

(72) Erfinder : **Roscher, Günter, Dr.**
**Altkönigstrasse 7**
**W-6233 Kelkheim (Taunus) (DE)**
Erfinder : **Pressler, Wilfried, Dr.**
**Am Flachsland 15**
**W-6233 Kelkheim (Taunus) (DE)**

EP 0 006 613 B2

Jouve, 18, rue Saint-Denis, 75001 PARIS

**Beschreibung**

Die Erfindung betrifft ein Verfahren zur Herstellung von N,N'-Diacetylethylendiamin (DAED) aus Ethylendiamin und Essigsäure.

Diese Reaktion verläuft unter Wasserabspaltung nach der Gleichung

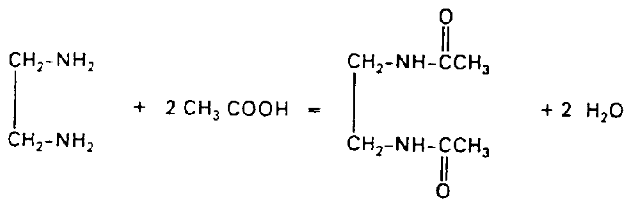


Aus Beispiel 1 der SU-PS 180 605 (vgl. Chem. Abstracts 69, 36131 g [1968] ist es bekannt, zur Herstellung von DAED Ethylendiamin mit mindestens einer stöchiometrischen Menge Essigsäure (als Eisessig oder als 50- bis 95%ige wässrige Lösung) bei erhöhter Temperatur unter Abdestillieren von Wasser umzusetzen.

Aus der DE-AS 21 18 282 ist bekannt, dass man DAED kontinuierlich herstellen kann, indem man Ethylendiamin und Essigsäure im Gewichtsverhältnis 1:2 bis 3 einer Schmelze von DAED bei einer Temperatur von 140 bis 215°C kontinuierlich zuführt, fortlaufend die überschüssige Essigsäure und das entstehende Wasser abdestilliert und das gebildete DAED in dem Masse entnimmt, wie es gebildet wird.

Aus der DE-AS 21 33 458 ist ferner bekannt, dass bei diskontinuierlicher Herstellung von DAED aus Ethylendiamin und Essigsäure im Gewichtsverhältnis 1:2 bis 2,5 die Bildung eines möglicherweise cyclischen Nebenprodukts unterdrückt werden kann, wenn man die Entfernung des Wassers erst bei einer Alkalität des Reaktionsgemisches von 50-10% des anfänglichen Werts einleitet.

Beim Einsatz von stöchiometrischen Mengen an Essigsäure (d.h. 2 Mol pro Mol Ethylendiamin) entsteht jedoch kein einheitliches Produkt (siehe Vergleichsbeispiel). Stattdessen destilliert Essigsäure mit dem gebildeten Reaktionswasser ab und das zurückbleibende Reaktionsprodukt reagiert alkalisch. Um reines DAED zu erhalten, muss man daher mit einem Überschuss an Essigsäure arbeiten. Die beiden obengenannten deutschen Auslegeschriften 21 18 282 und 21 33 458 bezeichnen daher auch den Einsatz eines Essigsäure-Überschusses als bevorzugt. Wenn man nun Ethylendiamin mit einer überstöchiometrischen Menge Essigsäure versetzt, muss man nach Beendigung der Reaktion die überschüssige Essigsäure wieder vom gebildeten DAED abdestillieren, und diese Essigsäure ist zwangsläufig durch das gleichzeitig überdestillierende Reaktionswasser verdünnt.

Die Wiedergewinnung von konzentrierter Essigsäure durch destillative Abtrennung des Wassers ist zwar möglich, jedoch mit hohem technischem Aufwand verbunden (vgl. Ullmanns Encycl. der techn. Chemie, Band 11 (1976) Seite 65). Der Erfindung liegt die Aufgabe zugrunde, DAED aus Ethylendiamin und Essigsäure so herzustellen, dass die zur Gewinnung eines reinen Produktes erforderliche überschüssige Essigsäure unter Umgehung einer solchen aufwendigen Essigsäure-Wasser-Trennung zurückgeführt werden kann. Das erfindungsgemäße Verfahren zur Herstellung von N,N'-Diacetylethylendiamin aus Ethylendiamin und Essigsäure ist dadurch gekennzeichnet, daß man

a) Ethylendiamin mit der stöchiometrisch notwendigen Menge Essigsäure versetzt, die zumindest teilweise in Form von wäßriger Essigsäure zugegeben wird ;

b) das in diesem Neutralisationsgemisch enthaltene Wasser zumindest teilweise bei 60-140°C abdestilliert ;

c) das zurückbleibende Neutralisationsgemisch mit überschüssiger Essigsäure versetzt;

d) auf 140-220°C erhitzt und das dabei gebildete Reaktionswasser, gegebenenfalls mit beim Verfahrensschrift b) nicht abdestilligform Wasser und gegebenenfalls noch mit wenig Wasser, welches die im Verfahrensschrift c) zugesetzte Essigsaüre möglichenweise enthielt, in Form einer wäßrigen Essigsäure abdestilliert und das als Sumpfprodukt anfallende N,N'-Diacetylethylendiamin abzieht;

e) die abdestillierte wäßrige Essigsäure in Shritt a) zurückführt.

In Schritt a) wird auf jeden Fall die in Schritt d) des vohergehenden Durchgangs abdestillierte verdünnte Essigsäure wiedereingesetzt. Außerdem wird soviel frische Essigsäure zugegeben, daß insgesamt die stöchiometrisch notwendige Menge Essigsäure vorliegt. Die frische Essigsäure kann in verdünnter oder konzentrierter Form zugeführt werden, bevorzugt verwendet man jedoch die in der Technik übliche, im wesentlichen wasserfreie Essigsäure.Das Ethylendiamin wird ebenfalls vorzugsweise in technisch wasserfreier Form eingesetzt. Die "stöchiometrisch notwendige"Menge Essigsäure sind dabei 2 Mol pro Mol Ethylendiamin.

Da in Schritt a) nur die stöchiometrisch notwendige Menge Essigsäure eingesetzt wird, beinhaltet das Abdestillieren des Wassers in Schritt b) keine Essigsäure-Wasser-Trennung, denn die Essigsäure ist an das Ethylendiamin gebunden und destilliert daher nicht über. Das erwähnte Wasser stammt 1. aus der zurückgeführten wäßrigen Essigsäure, die das in Schritt d) des vorhergehenden Durchgangs gebildete Reaktionswasser enthält, und 2. aus der verdünnten frischen Essigsäure, falls solche in Schritt a) verwendet wird. Vorzugsweise wird man natürlich in Schritt b) das in dem Neutralisationsgemisch enthaltene Wasser möglichst weitgehend abdestillieren, um eine Anreicherung von Wasser im Reaktionsgemish zu vermeiden. Es ist aber nicht unbedingt notwendig,das gesamte Wasser abzudestillieren, sondern man kann eine kleine Menge im Neutralisationsgemisch belassen, z.B. 1/2 Mol Wasser pro Mol Ethylendiamin, die dann im Kreis geführt wird. Das bedeutet, daß im Schritt b) eines Durchgangs vorzugsweise das gesamte Reaktionswasser (2 Mol pro Mol DAED) des vorhergehenden Durchgangs ( und ggf. außerdem mit wäßriger frischer Essigsäure in Schritt a) eingebrachtes Wasser) abdestilliert wird. Dabei wird eine Temperatur von 60 bis 140°C gewählt und Normaldruck oder Unterdruck angewandt.

In Schritt c) wird das nach dem Abdestillieren des Wassers zurückbleibende Neutralisationsgemisch mit überschüssiger Essigsäure versetzt. Die Essigsäure-Menge beträgt vorzugsweise 0, 1 bis 1 Mol pro Mol des in Schritt a) eingesetzten Ethylendiamins. Die Essigsäure sollte im allgemeinen möglichst wenig Wasser enthalten; vorzugsweise verwendet man die in der Technik übliche, im wesentlichen wasserfreie Essigsäure.

In Schritt d) wird auf 140 bis 220°C erhitzt, und die durch das dabei gebildete Reaktionswasser (und ggf. das in Schritt b) nicht abdestillierte restliche Wasser, sowie ggf. in Schritt c) mit der überschüssigen Essigsäure eingebrachtes Wasser) verdünnte überschüssige Essigsäure abdestilliert und am Sumpf das DAED abgezogen. Die verdünnte Essigsäure wird in Schritt a) zurückgeführt.

Das erfindungsgemässe Verfahren hat den Vorteil, dass man N,N′-Diacetylethylendiamin in hoher Ausbeute und sehr reiner Form erhält, ohne dass wässrige Essigsäure umständlich aufgearbeitet werden muss. Das erhaltene DAED kann ohne weitere Reinigung zum N,N,N′, N′-Tetraacetylethylendiamin umgesetzt werden, das als Waschmittelzusatz verwendet wird. Diese Umsetzung ist beispielweise in der US-PS 3.539.629 beschrieben.

Das erfindungsgemässe Verfahren lässt sich sowohl kontinuierlich als auch diskontinuierlich durchführen. Beim kontinuierlichen Verfahren verwendet man mindestens 2, vorzugsweise 3, hintereinandergeschaltete Reaktionszonen, z.B. eine Kaskade bestehend aus 3 Rührkesseln, und verfährt vorzugsweise wie folgt:

Im ersten Rührkessel wird Ethylendiamin mit frischer, technisch wasserfreier Essigsäure und wässriger Rückessigsäure bei 60-140°C im Gesamtmolverhältnis Ethylendiamin : Essigsäure von 1:2 neutralisiert, wobei die exakte Temperatur innerhalb dieses Bereiches so zu wählen ist, dass fortlaufend aus dem Neutralisationsgemisch pro Mol Ethylendiamin 2 Mol Wasser abdestillieren.

Das Sumpfprodukt wird kontinuierlich in einen zweiten Rührkessel übergeführt. Hier wird unter kontinuierlicher Zufuhr von 0,1 bis 1 Mol Essigsäure, vorzugsweise 0,3 bis 0,5 Mol, pro Mol eingesetztes Ethylendiamin durch Erhitzen auf 140-180°C, vorzugsweise 150-170°C, die Wasser-abspaltung begonnen. Sie wird in einem dritten Rührkessel bei 180-220°C, vorzugsweise bei 190-210°C, zu Ende geführt. Aus den Rührkesseln 2 und 3 destilliert man kontinuierlich das Reaktionswasser mit der überschüssigen Essigsäure ab. Die erhaltene wässrige Essigsäure wird in den ersten Rührkessel zurückgeführt. Das reine N,N′-Diacetylethylendiamin wird kontinuierlich als Schmelze aus dem dritten Rührkessel entnommen und erstarrt beim Erkalten. Man kann jedoch auch eine Kaskade aus mehr als 3 Rührkesseln benutzen.

Diskontinuierlich führt man das erfindungsgemässe Verfahren vorzugsweise so durch, dass man in einem Rührkessel das Gemisch aus frischer, technisch wasserfreier Essigsäure und wässriger Rückessigsäure vorlegt und die stöchiometrische Menge Ethylendiamin (Molverhältnis Ethylendiamin : Gesamtessigsäure 1:2) zugibt. Durch die Neutralisationswärme erwärmt sich die Mischung. Anschliessend werden 2 Molteile Wasser pro Mol Ethylendiamin bei Normaldruck oder im Vakuum bei 60 bis 140°C abdestilliert.

Dann werden 0,1 bis 1 Mol, vorzugsweise 0,4 bis 0,6 Mol Essigsäure pro Mol Ethylendiamin zugeführt. Anschliessend wird auf 140-220°C, bevorzugt 160-200°C aufgeheizt und das Reaktionswasser mit der überschüssigen Essigsäure bei Normaldruck oder im Vakuum abdestilliert. Die so erhaltene wässrige Essigsäure wird in den nächsten Ansatz wieder eingesetzt. Als Sumpf verbleibt reines praktisch farbloses N,N′-Diacetylethylendiamin.

Sowohl bei der kontinuierlichen als auch bei der diskontinuierlichen Arbeitsweise ist es zweckmässig, in allen Reaktionsgefässen Inertgas, z.B. Stickstoff, als Schutzgas zu verwenden.

Die Erfindung wird durch die folgenden Beispiele näher erläutert.

Beispiel 1 (Kontinuierliche Durchführung)

In einer Kaskade aus 3 Rührgefässen mit jeweils zwei Litern Inhalt, gibt man in das erste Rührgefäss stündlich 900 g (15 Mol) technisch wasserfreies Ethylendiamin (Reinheit > 99%), 1500 g (25 Mol) technisch wasserfreie Essigsäure (Wassergehalt < 0,1%) und 910 g 33%ige wässrige Rückessigsäure (300 g - 5 Mol reine Essigsäure). Im ersten Rührgefäss wird eine Temperatur von 135 °C eingestellt, dabei destillieren stündlich 535 g Wasser ab, die weniger als 1 Gew.-% Essigsäure enthalten.

Der Überlauf des 1. Rührgefässes wird in das 2. Rührgefäss geleitet, dem zusätzlich stündlich 360 g (6 Mol) technisch wasserfreie Essigsäure zugeführt werden, wobei eine Temperatur von 165 °C eingestellt wird. Das abdestillierende Essigsäure-Wasser-Gemisch wird in einer Vorlage aufgefangen.

Der Überlauf des 2. Rührgefässes wird in das 3. Rührgefäss geleitet, aus dem bei 195°C das restliche Essigsäure-Wasser-Gemisch abdestilliert wird, das ebenfalls in der genannten Vorlage aufgefangen wird.

Man erhält in dieser Vorlage stündlich 910 g 33%ige wässrige Essigsäure, die in das 1. Rührgefäss zurückgeführt wird. Aus dem Überlauf des 3. Rührgefässes erhält man stündlich 2225 g N,N′-Diacetylethylendiamin, das noch 2,5 Gew.-% Essigsäure und 0,5 Gew.-% Wasser enthält. Das entspricht einer quantitativen Ausbeute. Das erstarrte Produkt ist praktisch farblos und hat einen Schmelzpunkt von 174 °C.

Beispiel 2 (Diskontinuierliche Durchführung)

In einem Rührgefäss werden 1860 g (31 Mol) technisch wasserfreie Essigsäure (Wassergehalt < 0,1%) und 1350 g 40%ige wässrige Rückessigsäure (540 g = 9 Mol reine Essigsäure) vorgelegt. Anschliessend werden 1200 g (20 Mol) technisch wasserfreies Ethylendiamin (Reinheit > 99%) innerhalb von 30 Minuten zugegeben. Dabei erwärmt sich die Reaktionsmasse auf 125°C und es tritt Rückflusssieden ein. Anschliessend wird zunächst langsam der Druck bis auf 80 mbar reduziert und bei Sumpftemperaturen von 75 bis 105°C 700 g Wasser mit einem Essigsäuregehalt von weniger als 1 Gew.-% abdestilliert. Danach werden 600 g (10 Mol) technisch wasserfreie Essigsäure zugeführt. Bei 80 mbar wird langsam bis auf 175°C aufgeheizt und das entstehende Reaktionswasser mit der überschüssigen Essigsäure abdestilliert. Nach 3 Stunden sind 1350 g 40%ige wässrige Essigsäure abdestilliert, die in den nächsten Ansatz wieder eingesetzt wird. Als Sumpf verbleiben 2960 g N,N′-Diacetylethylendiamin, das noch 2 Gew.-% Essigsäure und 1 Gew.-% Wasser enthält. Das entspricht einer quantitativen Ausbeute. Das erstarrte Produkt ist praktisch farblos und hat einen Schmelzpunkt von 172 °C.

Vergleichsbeispiel (Einsatz der stöchiometrischen Menge Essigsäure)

In einem Rührgefäss werden 2400 g (40 Mol) technisch wasserfreie Essigsäure (Wassergehalt < 0,1%) vorgelegt. Anschliessend werden 1200 g (20 Mol) technisch wasserfreies Ethylendiamin innerhalb von 60 Minuten zugegeben. Dabei erwärmt sich die Reaktionsmasse auf 140°C und es tritt Rückflusssieden ein. Anschliessend wird noch 30 Minuten unter Rückflusssieden gerührt und danach das Reaktionswasser bei einer Sumpftemperatur bis 180 °C abdestilliert. Zur vollständigen Wasserentfernung wird bei dieser Temperatur langsam der Druck bis auf 80 mbar reduziert. Nach 3,5 Stunden sind insgesamt 710 g Wasser abdestilliert, das 4 Gew.-% Essigsäure enthält.

Als Sumpf verbleiben 2890 g rohes N,N′-Diacetylethylendiamin, das noch 1 Gew.-% Wasser enthält. Das erstarrte Produkt beginnt bereits bei ca. 90°C zu schmelzen; eine wässrige Lösung des Produkts reagiert alkalisch.

## Patentansprüche

1. Verfahren zur Herstellung von N,N′-Diacetylethylendiamin aus Ethylendiamin und Essigsäure, dadurch gekennzeichnet, daß man

   a) Ethylendiamin mit der stöchiometrisch notwendigen Menge Essigsäure versetzt, die zumindest teilweise in Form von wäßriger Essigsäure zugegeben wird ;

   b) das in diesem Neutralisationsgemisch enthaltene Wasser zumindest teilweise bei 60-140°C abdestilliert;

   c) das zurückbleibende Neutralisationsgemisch mit überschüssiger Essigsäure versetzt;

   d) auf 140-220°C erhitzt und das dabei gebildete Reaktionswasser, gegebenenfalls mit beim Verfahrensschrift b) nicht abdestilligform Wasser und gegebenenfalls noch mit wenig Wasser, welches die im Verfahrensschrift c) zugesetzte Essigsäure möglichenweise enthielt, in Form einer wäßrigen Essigsäure abdestilliert und das als Sumpfprodukt anfallende N,N′-Diacetylethylendiamin abzieht;

   e) die abdestillierte wäßrige Essigsäure in Schritt a) zurückführt.

## Claims

1. A process for the preparation of N,N′-diacetylethylenediamine from ethylenediamine and acetic acid, which comprises
   a) adding the stoichiometrically required amount of acetic acid at least partially in the form of aqueous acetic acid to ethylenediamine;
   b) distilling off, at 60-140°C, at least partially the water contained in this neutralization mixture;
   c) adding excess acetic acid to the remaining neutralization mixture;
   d) heating the mixture to 140-110°C and distilling off the water formed in the reaction, with any water not distilled off in process step b) and possibly also with a small quantity of water which the acetic acid added in process step c) may have contained, in the form of an aqueous solution of acetic acid, and withdrawing the N,N′-diacetylethylenediamine obtained as a bottom product;
   e) recycling the aqueous acetic acid distilled off into step a).

## Revendications

1. Procédé de préparation de la N,N′-diacétyl-éthylène-diamine à partir de l'éthylène-diamine et de l'acide acétique, procédé caractérisé en ce que :
   a) on ajoute à l'éthylène-diamine la quantité d'acide acétique stoechiométriquement nécessaire, au moins en partie sous la forme d'acide acétique aqueux,
   b) on chasse par distillation, au moins partiellement, l'eau contenue dans ce mélange de neutralisation, à une température de 60 à 140°C
   c) on ajoute au mélange de neutralisation qui reste un excès d'acide acétique,
   d) on chauffe à une température de 140 à 220°C et on chasse par distillation l'eau engendrée par la réaction -éventuellement avec l'eau qui n'a pas été chassée par distillation lors de l'étape opératoire b) et éventuellement avec un peu d'eau contenue, le cas échéant, dans l'acide acétique ajouté lors de l'étape opératoire c)- sous la forme d'un acide acétique aqueux, et on retire la N,N′-diacétyl-éthylène-diamine constituant le produit du fond,
   e) on renvoie à l'étape a) l'acide acétique aqueux qui s'est échappé par distillation.